# EUROPEAN PATENT SPECIFICATION

(11) **EP 2 943 147 B1**
(45) Date of publication and mention of the grant of the patent: **27.09.2017**
(21) Application number: 14700291.9
(22) Date of filing: 10.01.2014
(51) Int. Cl.: A61F 2/00

(54) **SURGICAL IMPLANT FOR THE TREATMENT OF THE PROLAPSE OF A PELVIC ORGAN BY LAPAROSCOPY VIA SACROCOLPOPEXY AND/OR SACROHYSTEROPEXY**
CHIRURGISCHES IMPLANTAT ZUR BEHANDLUNG EINES BECKENORGANPROLAPS DURCH SAKROKOLPOPEXIE UND/ODER SAKROHYSTEROPEXIE
IMPLANT CHIRURGICAL POUR LE TRAITEMENT D'UN PROLAPSUS D'ORGANE PELVIEN PAR SACROCOLPOPEXIE ET/OU SACROHYSTÉROPEXIE

(30) Priority: 10.01.2013 EP 13382005
(43) Date of publication of application: 18.11.2015
(73) Proprietor: B. Braun Surgical, S. A., 08191 Rubi (ES)
(72) Inventor: NIEVES MARTIN, Tania, 08191 Rubi (ES); WEIS, Christine, 08191 Rubi (ES); TURON DOLS, Pau, 08191 Rubi (ES); DEL CASTILLO RIESTRA, Luis Felipe, 08191 Rubi (ES)
(74) Representative: Oficina Ponti, SLP
(86) International application number: PCT/EP2014/050361
(87) International publication number: WO 2014/108493

(56) References cited:
- EP-A1- 2 340 784
- WO-A1-2007/118260
- FR-A1- 2 976 788
- US-A1- 2010 305 394
- US-A1- 2011 105 836
- US-A1- 2011 297 161

## Description

The present invention relates to an implant for the surgical repair of the pelvic floor by laparoscopy which adjusts to the techniques called sacrocolpopexy and/or sacrohysteropexy. In particular, the present invention relates to an implant for the treatment of vaginal vault prolapse or uterine prolapse using said techniques.

### Background of the invention

Uterine prolapse or vaginal vault prolapse is a disorder of the pelvic floor organs produced by different causes that give rise to numerous problems such as, for example, pelvic pain, problems in sexual relations, it may affect urinary incontinence, etc. decreasing the quality of life of the person suffering from it.

Vaginal vault prolapse can be treated using the surgical technique called colposacropexy or sacrocolpopexy and uterine prolapse can be treated by the surgical technique called sacrohysteropexy. These techniques entail suspension of the vagina or uterus to a point of the pelvic cavity, preferably, to the patient's sacral promontory, via a surgical implant which typically consists of a fabric of implantable polymeric material in the form of mesh. The objective of the mesh is to replace the displaced fascias and substitute the damaged native tissue.

The sacrocolpopexy technique and the sacrohysteropexy technique can be performed via an abdominal incision, although it is currently performed by laparoscopy since this technique is less aggressive for the patient and also offers numerous advantages. In particular, experts consider that laparoscopy is the only technique that makes it possible to repair the double angle of the vagina (posterior and anterior angle) with a constant vision and direct access to the deepest areas of the pelvis, achieving a suitable and lasting securing of the vagina to the sacral promontory through a surgical mesh.

Patents WO2010/138894, WO2010/87923 and WO02/19946 disclose implantable fabrics in the form of mesh which have the special characteristic that all have a general Y-shaped configuration. This configuration includes a first portion for the fastening of the fabric to the sacral promontory and a second portion connected to the first portion for the fastening of the fabric to the pelvic organ. The second portion includes two superimposed sections of fabric to respectively fix in the anterior and posterior part of the pelvic organ. These two sections of fabric have the special characteristic that they are joined together by way of a pin, each one of the sections determining one arm of said pin.

Patent document US2011/297161 refers to a method of treating prolapse of a vagina including the step of securing a base of a Y- shaped support to tissue by inserting an anchor into the tissue, the anchor coupled to the Y-shaped support via an interconnecting member, the step of asymmetrically securing the base of a Y-shaped support to one anterior sacroiliac ligament connected to and located alongside of the sacrum bone and securing each of a pair of legs of the Y-shaped support to opposing sides of the vagina, and the final step of adjusting elevation of the cuff of the vagina relative to the sacrum bone by sliding the interconnecting member relative to the adjustable anchor.

The Y-shaped implantable meshes or fabrics of the patent documents mentioned have the drawback that they are difficult to handle for the surgeon, especially when the operation is carried out by laparoscopy which entails the use of a trocar introduced in the abdomen.

In particular, it has been observed that the Y-shaped implantable meshes or fabric have joints or welds in the points of intersection of the arms or sections that define the fastening pin. These joints or welds mean that the mesh is less malleable when being introduced in the trocar cannula, which entails more work for the surgeon and, furthermore, in some cases, the risk of tearing the mesh.

Another drawback of the meshes or fabrics disclosed in said patents lies in the fact that to be able to fix the mesh to the anterior and posterior part of the pelvic organ it is necessary to separate the two arms or sections of superimposed fabric that define the pin. However, this operation is not at all easy to carry out through the trocar, which again entails more work for the surgeon.

On the other hand, it is important to highlight that a fundamental principal in the practice of sacrocolpopexy is that the fixing of the mesh or fabric to the sacral promontory must be performed without tension, since the tensions are translated into problems of pain in the patient. To eradicate these tensions it is essential to ensure a correct distribution of forces in the organ.

Patent WO2007/118260 discloses an implantable mesh for the surgical reconstruction in the area of the pelvic floor including an anterior mesh segment between the bladder and the vagina, a posterior mesh segment between the vagina and the rectum, a pair of distal transobturator holding straps extending from the anterior mesh segment, a pair of proximal transobturator holding straps extending from the anterior mesh segment and a pair of lower dorsal holding straps extending from the posterior mesh segment. According to this patent document, the mesh further includes an intermediate segment located between the anterior mesh segment and the posterior mesh segment. The pair of lower dorsal holding straps extends from the region of the posterior mesh segment bordering on the intermediate segment and a pair of upper dorsal holding straps extends from the region of the anterior mesh segment bordering on the intermediate segment.

Patent document US2011/105836 discloses a system and method of treating vaginal prolapse and incontinence comprising a kit. The kit includes a mesh graft configured for Attachment to the anterior and posterior vaginal walls to thereby treat the vaginal prolapse. A graft delivery device is also provided for introducing and placing the mesh graft to a location deep within the peritoneal cavity and for attaching the graft thereto. A leg assembly is provided and coupled to an end of the mesh graft and cooperates with the graft delivery device to anchor and affix the mesh graft to the desired anatomical structures.

Patent document FR 2976788 discloses a mesh design for sacrocolpopexy procedure that has a configuration for covering the vagina and an extension to the sacrum to provide for apical support.

Patent EP 2340784 discloses a textile mesh, in particular for pelvic floor reconstruction or for preventing dyspareunia, comprising a central mesh section and two lateral mesh sections on both sides of the central mesh section.

It has been observed that the Y-shaped fabrics or meshes of the documents of the state of the art do not guarantee the desired distribution of forces, unwanted folds forming in many cases during its placement which constitute, furthermore, a risk factor for infections and possible erosions.

### Description of the invention

The objective of the present invention is to resolve said drawbacks, developing a surgical implant for the treatment of the prolapse of a pelvic organ by laparoscopy and via sacrocolpopexy and/or sacrohysteropexy which resolve the aforementioned problems and have the advantages described below.

In accordance with this objective, according to a first aspect, the present invention provides a surgical implant for the treatment of the prolapse of a pelvic organ by laparoscopy and via sacrocolpopexy and/or sacrohysteropexy, comprising a fabric of implantable material including;
a first portion for the fastening of said fabric to one or more points of the pelvic cavity and,
a second portion which is joined to said first portion for the fastening of said fabric to said pelvic organ, said second portion of fabric including;
two sections of fabric to respectively fix in the anterior and posterior part of said pelvic organ, said two sections of fabric being disposed substantially parallel in the same plane and separated from one another by a distance "d", said second portion of fabric further comprising;
a third section of fabric which acts as connecting bridge of said two sections to cover said distance "d" of separation, wherein said third section of fabric which act as a bridge extends between said two parallel sections with its lateral edge substantially aligned with the edge of the ends of said two parallel sections, toward the first portion and wherein said first portion for the fastening of the fabric to the pelvic cavity includes;
a section of fabric to fix to the sacral promontory which extends substantially aligned with and joined to the end of one of said two parallel sections, said section of fabric to fix to the sacral promontory forming a substantially right angle with the third section which acts as a bridge, making it possible for said third bridge section to partially and laterally surround said pelvic organ to place said two parallel sections in the anterior or posterior part of said organ.

The surgical implant of the present invention has numerous advantages with respect to the implants of the state of the art. In particular, the implant is much easier to place, since it has two sections of fabric to fix to the pelvic organ which are situated in the same plane and are separated from one another by a pre-established distance "d". This distance "d" between the sections of fabric situated in the same plane makes it easier for the surgeon to position the implant, providing him with a much greater margin of manoeuvre that those provided by the pin of the Y-shaped implants of the state of the art. Indeed, in the implants of the state of the art, the surgeon is obliged to separate the sections of superimposed fabric of the pin to place the implant, meaning that the manoeuvre is much more complicated and slower, especially when the operation is carried out by laparoscopy.

Another advantage of the implant claimed lies in the fact that it has a third section of fabric which acts as a bridge between the two sections mentioned to cover said distance "d" and comfortably and laterally surround the organ. This third bridge section provides an additional fastening coverage of the pelvic organ and enables, on the other hand, the safe and comfortable treatment of vaginal prolapse that conserves the uterus.

In the implants of the state of the art, the treatment of uterine or vaginal prolapse which conserves the uterus is very difficult since the adjustment of the arms of the pin around the vagina and the fastening to the sacrum is very complex, for which reason, in practice, these Y-shaped implants are only used in the sacrocolpopexy technique. Other known implants, as the one described in patent WO2007/118260, likewise do not enable comfortable treatment of a vagina in a patient that conserves the uterus since in this case an intermediate segment of the implant is designed to be folded over the prolapsed vagina.

Another advantage of the implant claimed lies in the fact that it has the advantage that it can be fixed by laparoscopy to the pelvic organ without tensions. This is due to the fact that as the sections of fabric are situated in the same plane, it is easier to adjust the implant to the anatomy of the pelvic organ without problems, avoiding the formation of folds and guaranteeing at all times a correct distribution of forces in the organ.

The implant of the present invention has a configuration substantially in the form of a chair which has the advantage that it is very easy for the surgeon to handle and substantially reduces the formation of folds or creases. Indeed, as the fixing section to the sacrum and one of said parallel fixing sections to the pelvic organ are aligned, there are fewer steps to be performed by the surgeon to place the implant and the adaptation to the organ's anatomy is easier.

Preferably, said first and second portion of fabric are integrated in a single piece of fabric of substantially flat structure without welds or joints. Furthermore, advantageously, the structure and density of said fabric is uniform for all the sections mentioned of the implant.

In this way, a very malleable fabric is obtained which is particularly interesting for use in laparoscopy since it can be coiled and uncoiled without problems as it does not have welds or joints which make the implant more rigid. On the other hand, as it is a fabric with uniform structure and density, it favours a better distribution of the tensile forces in the fixing points.

Advantageously, according to the same preferred embodiment, said section of fabric to fix to the sacrum extends from the end of one of said parallel sections maintaining one of its lateral edges substantially aligned with the lateral edge of the section whereto it is joined.
Preferably, the distance "d" of separation between said two parallel sections of fabric, or the width of the section acting as a bridge, is dimensioned to laterally surround the pelvic organ to enable the safe and comfortable treatment of vaginal prolapse that conserves the uterus. Advantageously, said distance "d" ranges between 0.035 m (35mm) and 0.045 m (45 mm).

It has been observed that this distance is suitable to connect the anterior and posterior area of a pelvic organ such as, for example, the vagina to repair the vaginal and/or uterine prolapse.

Again preferably, said two parallel sections of fabric are each configured in the form of a rectangle with suitable dimensions to anatomically cover the anterior or posterior part of said pelvic organ.

Advantageously, if said pelvic organ is a vagina, one of said parallel sections in the form of a rectangle has an area of magnitude greater than that of the other section to anatomically cover the anterior part of said vagina.

Again advantageously, said section of fabric to fix to the sacral promontory is configured in the form of a rectangle with a width less than the width of the section of fabric of the second portion of the implant whereto it is joined.

According to a preferred embodiment, said fabric of implantable material is a mesh, preferably, a mesh of monofilament polypropylene yarns classified within group I of the AMID classification.

This mesh may be composed of a biological material, such as, for example, collagen, or of a mixture of a biological and synthetic material. Furthermore, the mesh may contain antimicrobial agents.
Advantageously, the pores of said mesh are of a size equal to or greater than 0.0010 m (1 mm) and, preferably, the geometric structure of said pores is substantially regular, such as, for example, a geometric structure in the form of a hexagon. Nevertheless, this geometric structure can also be irregular.

The pore size claimed facilitates the integration of the mesh. On the other hand, its substantially regular pore structure allows that the tensile forces and elongation of the fabric in the longitudinal and transversal directions are very similar. This detail reduces the appearance of tensions with the biological fabrics.

According to one preferred embodiment, the implant comprises a section of fabric to fix to the sacral promontory, a section of fabric to fix to the posterior part of the pelvic organ, a section of fabric to fix to the anterior part of the pelvic organ, said two sections of fabric to fix to the anterior and the posterior part of the organ being disposed substantially parallel in the same plane and separated from one another by a distance "d", said implant further to cover said distance "d", said bridge section being dimensioned and configured to laterally surround said pelvic organ to enable the safe and comfortable treatment of vaginal prolapse that conserves the uterus.

The dimensions of the four sections of the fabric comprising the implant may vary in accordance with the dimensions of the pelvic organ to be treated. Nevertheless, these dimensions will preferably be those stated below, where "A" corresponds to the width and "h" corresponds to the length of each one of the sections, hereinafter, sections 3a, 5a, 5b and 5c.

### Section 3a to fix to the sacral promontory

A₃ₐ= between 0.020 m (20 mm) and 0.040 m (40 mm)
h₃ₐ= between 0.080 m (80 mm) and 0.120 m (120mm)

### Section 5a to fix to the posterior part of the pelvic organ:

A₅ₐ = between 0.030 m (30mm) and 0.050 m (50 mm)
h₅ₐ= 0.070 m (70 mm) and 0.110 m (110 mm)

### Section 5b to fix to the anterior part of the pelvic organ:

A_{5b}= between 0.040 m (40 mm) and 0.060 m (60 mm)
h_{5b}= 0.080 m (80 mm) and 0.120 m (120 mm)

### Section 5c which acts as a bridge and surrounds the pelvic organ:

A_{5c}= between 0.035 m (35 mm) and 0.045 m (45 mm), this dimension corresponding to the distance "d" claimed.
h_{5c}= between 0.010 m (10 mm) and 0.050 m (50 mm)
It is here also described a method to treat the prolapse of a pelvic organ, preferably by laparoscopy, via the surgical implant claimed comprising the stages of;
a) situating one of the two sections of fabric of the second portion of the implant in the posterior or anterior area of the pelvic organ,
b) situating another of the two sections of fabric of the second portion of the implant in the anterior or posterior area of the pelvic organ, partially and laterally surrounding for this the pelvic organ with the third bridge section of fabric of the implant,
c) fixing the two sections of fabric respectively to the anterior and posterior area of the pelvic organ and, optionally, also fixing the third bridge section of fabric to the vaginal vault when said organ is a vagina, and
d) later, fixing the first portion of the implant to a point of the pelvic cavity, preferably, to the sacral promontory of the patient.
Advantageously, before stage a), the step of opening the peritoneum is performed in the right side of the pelvic cavity, preferably, in the right side of the sacral promontory, said opening allowing the introduction of the implant within the abdomen through a laparoscopic trocar. Again advantageously, in stage a), a section of fabric of the second portion is positioned in the posterior area of the pelvic organ and, subsequently, in stage b), the other section of fabric of the second portion is positioned in the anterior area of the same pelvic organ.

The method to treat the prolapse of a pelvic organ by the implant claimed is very fast and comfortable, since the surgeon can laterally surround the pelvic organ with the implant to situate the position of the fixing arms or sections. In the implants of the state of the art, this manoeuvre is not possible since the arms or sections of the pin are superimposed.

In accordance with a second aspect, the present invention relates to a surgical kit comprising the implant claimed, a laparoscopic instrument to introduce the implant and means to fix the position of the implant in the organ once placed.
Said means to fix the position of the implant may comprise absorbable or non-absorbable sutures and/or "tackers", or biocompatible adhesive, for example an adhesive including fibrin or cyanoacrylates, etc...

The implant claimed has the advantage that it makes viable and facilitates the use of the adhesive on the parts of the organ thanks to the additional coverage provided by the sections of the second portion of the implant. This adhesive can be applied by a specially designed applicator for laparoscopic use.

In the present invention, pelvic organ is understood to be any organ of the pelvic cavity of a woman, preferably, a vagina or uterus.

### Brief description of figures

For a better understanding of all that disclosed, drawings are attached wherein, diagrammatically and only as a non-limitative example, one embodiment of the invention is represented.

### In said drawings,

Figures 1 and 2 show plan views of an embodiment of the implant. In these figures, the different sections of the implant's fabric have been delimited diagrammatically using broken lines to facilitate the description.
Figure 3 is a perspective view showing a vagina with uterus and the implant of the embodiment of the figure 1 designed to secure said vagina for the treatment of the prolapse.
Figure 4 is a perspective view showing the manner in which the surgeon situates the implant on the pelvic organ, in this case, a vagina.
Figure 5 is a perspective view showing the implant already disposed on the vagina.
Figure 6 is a perspective view showing the manner in which the implant is situated and secured inside the pelvic cavity.
Figure 7 is a section of anatomy showing the manner in which the implant is situated and secured inside the pelvic cavity.

### Description of a preferred embodiment

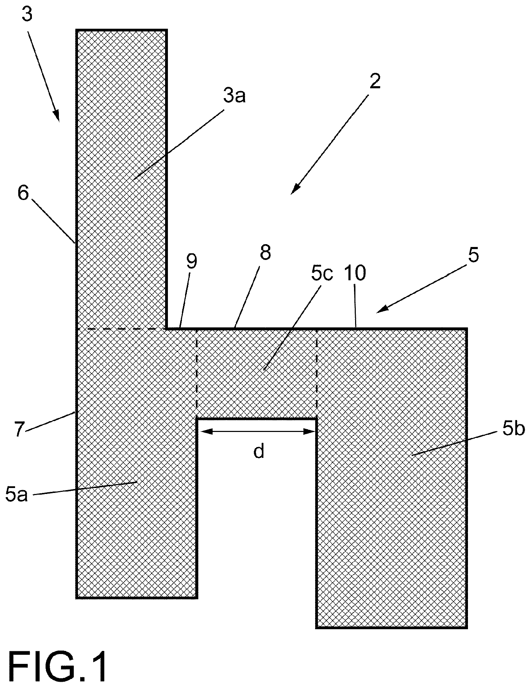

The implant of the present invention is described below, making reference to figures 1 to 7. These figures show an implant for the treatment of the prolapse of a vagina 1b which conserves the uterus 1a via sacrohysteropexy.

The implant claimed comprises a fabric 2 of implantable material which, in the embodiment described, is a mesh of uniform structure and density manufactured from monofilament polypropylene yarns and classified within group I of the AMID classification. The pore size of this mesh is between 0.0028 m (2.8 mm) and 0.0036 m (3.6 mm) and the geometric structure of the pore is regular in the form of a hexagon.

This fabric 2 in the form of mesh includes a first portion 3 for the fastening of the fabric 2 to a point of the pelvic cavity, in this case, the sacral promontory 4, and a second portion 5 for the fastening of the fabric 2 to the pelvic organ, in this case, the vagina 1b.

The second portion 5 of the fabric 2 comprises two sections 5a, 5b of fabric 2 in the form of a rectangle to respectively fix in the anterior and posterior part of the pelvic organ. As can be seen in figure 1, these two sections 5a, 5b of fabric 2 are disposed substantially parallel in the same plane and separated from one another by a distance "d". This same second portion 5 of fabric 2 includes a third section 5c of fabric 2 which acts as a connecting bridge of the parallel sections 5a, 5b to be able to surround the pelvic organ.

The first portion 3 of fabric 2 comprises a single section 3a of fabric 2 in the form of a rectangle to fix to the sacral promontory 4. This section 3a of fabric 2 extends aligned with section 5a of the second portion 5 maintaining one of its lateral edges 6 substantially aligned with the lateral edge 7 of said section 5a and forming a substantially right angle with the third section 5c of fabric 2 which acts as a bridge. On the other hand, the third section 5c which acts as a bridge extends between the two parallel sections 5a, 5b maintaining its upper lateral edge 8 substantially aligned with the upper edge 9,10 of the ends of said two sections 5a, 5b.

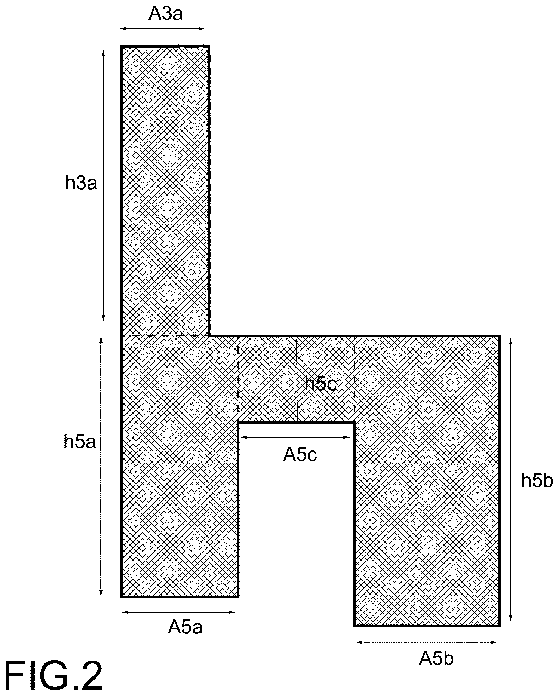

In this way, as can be seen in figures 1 and 2, the fabric 2 of the implant acquires a general chair-shaped configuration which has the advantage that it is very easy to handle for the surgeon and substantially avoids the formation of folds or creases, since it is easier to adapt to the patient's anatomy.

In the embodiment described, the four sections 3a, 5a, 5b, 5c of fabric 2 which define the implant are integrated in a single piece of fabric 2 of substantially flat structure without welds or joints. Thanks to this, the implant is highly malleable for which reason it is particularly interesting for use in laparoscopy, since it can be coiled and uncoiled and penetrate in the trocar without the risk of it breaking.

Intervention by laparoscopy has the following advantages in the repair of vaginal prolapse with uterus via the implant of the present invention:
▪ The vagina is situated in horizontal position with respect to the plane of the levators and its angle can be corrected, and, as the uterus is conserved, the fixing of the cervix in the sacral cavity is physiological.
▪ The fixing of the implant is safer and more stable.
▪ A complementary laparoscopic colposuspension can be performed (for example, according to Bursch's technique).
▪ As the uterus is conserved, there are high possibilities of pregnancy and low possibilities of miscarriage.
▪ Lack of scars and without lateralization of the vagina.
▪ Greater length of the vagina after fixing to the sacrum, especially in android type vaginas and where is a shorter distance between the introitus and the ischiatic spine, which conditions a short vagina.
▪ Capacity of re-assessing all defects in the same surgical act and fully repairing them after readjusting the therapeutic strategy.
A method to treat the prolapse of a pelvic organ via the surgical implant claimed is described below.

In the embodiment described of the implant, the pelvic organ to treat is a vagina 1b which conserves the uterus 1 a of approximately 0.08 m - 0.11 m (8- 11 cm), with an anterior area of approximately 0.07 m (7 cm) and a posterior area of approximately 0.09 m (9 cm). The uterus 1a has general anatomical measurements of 0.08 m (8 cm) in length, 0.05 m (5 cm) in width and 0.025 m (2.5 cm) thickness.

The dimensions of the fabric 2 of the implant used in this embodiment are the following (see figure 2):
- Section 3a to fix to the sacral promontory: A₃ₐ= 0.03 m (30 mm), h₃ₐ= 0.100 m (100 mm)
- Section 5a to fix to the posterior part of the vagina 1b: A₅ₐ = 0.04 m (40 mm), h₅ₐ= 0.09 m (90 mm)
- Section 5b to fix to the anterior part of the vagina 1b: A_{5b}= 0.050 m (50 mm), h_{5b}= 0.100 m (100 mm)
- Section 5c which acts as a bridge and surrounds the vagina 1b: A_{5c}= 0.040 m (40 mm), h_{5c}= 0.030 m (30 mm): The dimension A_{5c} corresponds to the distance "d" claimed of section 5c.

The fabric 2 used is a mesh composed of monofilament polypropylene fibres with the following (general) technical characteristics:
- Thickness of the fabric 2: 0.0005 m (0.5 mm)
- Weight: 0.048 Kg/m²
- Tensile force in longitudinal direction: equal to or greater than 25 N
- Tensile force in transversal direction: equal to or greater than 25N
- Elongation in longitudinal direction: equal to or greater than 60%
- Elongation in transversal direction: equal to or greater than 60 %
- Tear strength of suture in longitudinal direction: 31N
- Tear strength of suture in transversal direction: 35 N
- Diameter of the yarn: 0.000150 m (0.150 mm)
- Pore size of the mesh: macropore of 0.0030 m (3.0 mm)

### Description of the method of placement and fixing

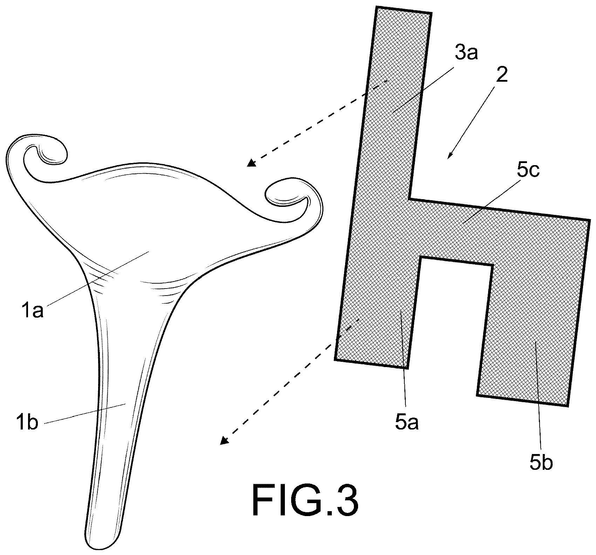

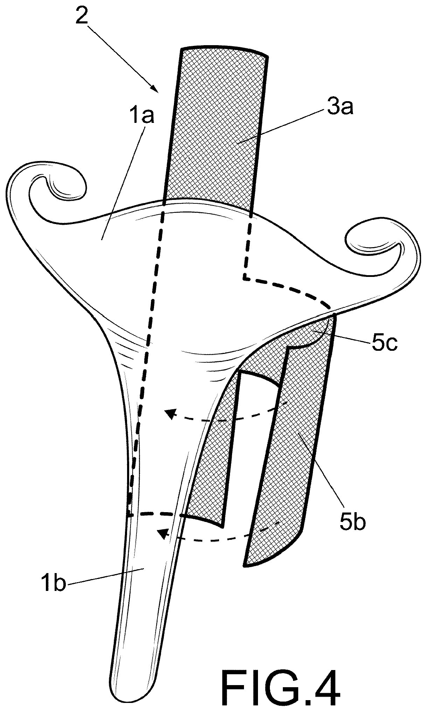

Firstly, section 5a of fabric 2 is situated in the posterior area of the vagina 1b (see figures 3 and 4). Therefore, the peritoneum is opened just in the right side of the sacral promontory 4. After this step has been performed, the mesorectum is dissected towards the right uterosacral ligaments. The retrorectal space must be found, but not the presacral space. Then, the implant is placed in the posterior area, i.e. section 5a is placed in the posterior part of the vagina 1b, in front of the rectum, and it is fixed to the sides of the space of the rectum levator, being secured in the lower posterior area of the vagina 1b.

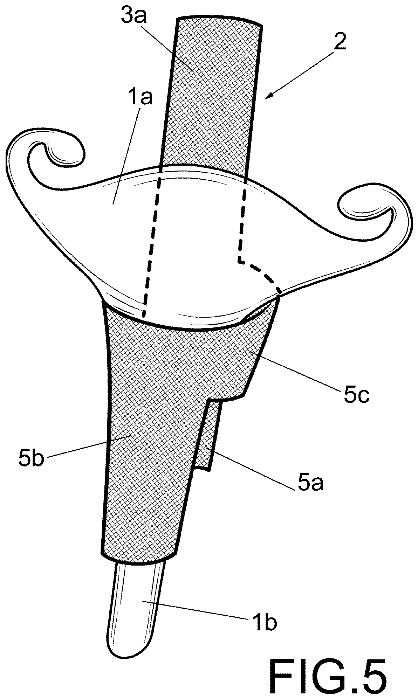

Next, once section 5a is placed and fixed, the vagina 1b is surrounded with section 5c of fabric 2 which acts as a bridge and section 5b is situated in the anterior area of the vagina 1, behind the rectum (see figure 4). Then, section 5b is fixed in the lower anterior area of the vagina 1b. The dimensions of this section 5b make it possible to anatomically cover the entire anterior area of the vagina 1b (see figure 5).

Once sections 5a and 5b are situated and placed in the posterior and anterior parts of the vagina 1b, the bridge section 5c is secured in the vaginal vault. Although this fastening is not strictly necessary, it is recommended to guarantee that the implant is completely fixed.

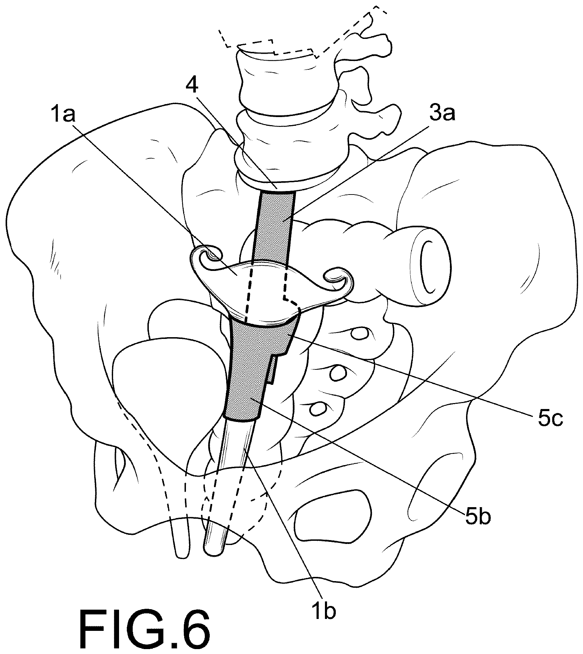

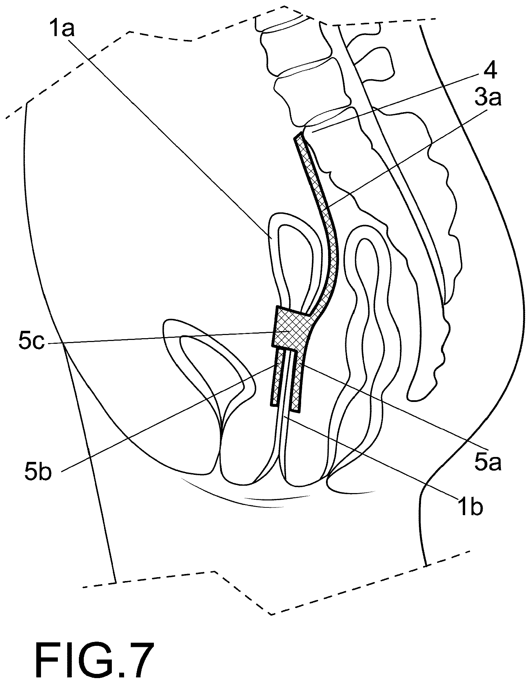

The last step of the treatment consists of fixing in distended form the section 3a of fabric 2 to the sacral promontory 4 of the patient. Figures 6 and 7 represent the implant situated and fixed in the organ of the patient suffering the prolapse, in this case, the vagina 1b.

As can be seen in these figures 6 and 7, the implant of the present invention enables a complete fastening that is much greater than that enabled by the implants of the state of the art. This greater coverage also guarantees a more uniform distribution of holding forces of the organ which avoids tensions, subsequent problems of pain and the appearance of recurrences. The composition and uniform geometric structure of the fabric 2, as well as its uniform mechanical properties both in longitudinal and transversal direction, contribute to improving the distribution of forces and favours the integration of the fabric 2 in the patient's body.

Despite the fact that reference has been made to a specific embodiment of the present invention, it is evident for a person skilled in the art that numerous variations and modifications can be made to the implant of the invention, without departing from the scope of protection defined by the attached claims.

## Claims

1. Surgical implant for the treatment of the prolapse of a pelvic organ (1a,1b) by laparoscopy and via sacrocolpopexy and/or sacrohysteropexy, comprising a fabric (2) of implantable material including;
- a first portion (3) for the fastening of said fabric to one or more points of the pelvic cavity,
- a second portion (5) which is joined to said first portion (3) for the fastening of said fabric (2) to said pelvic organ (1a,1b), said second portion (5) of fabric (2) including;
- two sections (5a,5b) of fabric (2) to respectively fix in the anterior and posterior part of said pelvic organ (1a,1b), said two sections (5a,5b) of fabric (2) being disposed substantially parallel in the same plane and separated from one another by a distance "d", said second portion (5) of fabric (2) further comprising;
- a third section (5c) of fabric which acts as connecting bridge of said two parallel sections (5a,5b) to cover said distance "d" of separation, wherein said third section of fabric which act as a bridge extends between said two parallel sections with its lateral edge substantially aligned with the edge of the ends of said two parallel sections, toward the first portion (3) and wherein
said first portion (3) for the fastening of the fabric to the pelvic cavity includes;
- a section (3a) of fabric (2) to fix to the sacral promontory which extends substantially aligned with and joined to the end of one of said two parallel sections (5a,5b), said section (3a) of fabric (2) to fix to the sacral promontory forming a substantially right angle with the third section (5c) which acts as a bridge, making it possible for said third bridge section (5c) to partially and laterally surround said pelvic organ (1a,1b) to place said two parallel sections (5a,5b) in the anterior or posterior part of said organ (1a, 1b).

2. Implant according to claim 1, wherein said first and second portion (3,5) of fabric (2) are integrated in a single piece of fabric (2) of substantially flat structure without welds or joints between said sections (3,5).

3. Implant according to claim 1, wherein the structure and density of said fabric (2) is uniform for all the sections (3,5a,5b,5c) of the implant.

4. Implant according to any of claims 1 to 3, wherein said section of fabric (3a) to fix to the sacrum (4) extends from the end of one of said parallel sections (5a) maintaining one of its lateral edges (6) substantially aligned with the lateral edge (7) of the section (5a) whereto it is joined.

5. Implant according to any of the preceding claims, wherein said distance "d" of separation, or the width (A_{5c}) of the third section which acts as a bridge (5c), is dimensioned to laterally surround the pelvic organ (1b) to enable the safe and comfortable treatment of vaginal (1b) prolapse that conserves the uterus (1a).

6. Implant according to claim 5, wherein said distance "d" ranges between 0.035 m and 0.045 m.

7. Implant according to any of the preceding claims, wherein said two parallel sections (5a,5b) of fabric (2) are each configured in the form of a rectangle with suitable dimensions (A₅ₐ, A_{5b}, h₅ₐ,h_{5b}) to cover the anatomy of the anterior or posterior part of said pelvic organ (1b).

8. Implant according to claim 7, wherein said pelvic organ is a vagina (1b) and one of said parallel sections (5b) in the form of rectangle has an area of magnitude greater than that of the other section (5a) to anatomically cover the anterior part of said vagina (1b).

9. Implant according to claim 7, wherein said section (3a) of fabric to fix to the sacral promontory (4) is configured in the form of a rectangle with a width (A₃ₐ) less than the width (A₅ₐ) of the section (5a) of fabric (2) of the second portion (3) of the implant whereto it is joined.

10. Implant according to claim 1, wherein said bridge section (5c) is dimensioned and configured to laterally surround said pelvic organ (1b) to enable the safe and comfortable treatment of vaginal (1b) prolapse that conserves the uterus (1a).

11. Implant according to claim 1, wherein said fabric (2) is a mesh, preferably, a mesh of monofilament polypropylene yarns classified within group I of the AMID classification.

12. Implant according to claim 11, wherein the pores of said mesh are of a size equal to or greater than 0.0010 m.

13. Implant according to any of claims 11 to 12, wherein the geometric structure of said pores is substantially regular, preferably, a geometric structure, in the form of a hexagon.

14. Implant according to any of the preceding claims, wherein said pelvic organ is a vagina (1b) or a uterus (1a) of a patient.

15. Surgical kit comprising an implant according to any of the preceding claims, a laparoscopic instrument to introduce the implant and means to fix the position of the implant in the organ once placed, said means preferably including a biocompatible adhesive to fix the sections (5a,5b,5c) of the second portion (5) of the implant on the pelvic organ (1a, 1b).

## Patentansprüche

1. Chirurgisches Implantat zur Behandlung des Prolapses eines Beckenorgans (1a, 1b) durch Laparoskopie sowie mittels sakraler Kolpopexie und/oder sakraler Hysteropexie, wobei das chirurgische Implantat ein Gewebe (2) aus einem implantierbaren Material aufweist, das folgende Merkmale umfasst:
- einen ersten Abschnitt (3) zum Anbringen des Gewebes an einem oder mehreren Punkten der Beckenhöhle,
- einen zweiten Abschnitt (5), der mit dem ersten Abschnitt (3) zum Anbringen des Gewebes (2) an dem Beckenorgan (1a, 1b) verbunden ist, wobei der zweite Abschnitt (5) des Gewebes (2) Folgendes umfasst:
- zwei Bereiche (5a, 5b) des Gewebes (2) zum jeweiligen Befestigen in dem vorderen beziehungsweise hinteren Teil des Beckenorgans (1a, 1b), wobei die zwei Bereiche (5a, 5b) des Gewebes (2) im Wesentlichen parallel in derselben Ebene angeordnet und voneinander durch eine Abstand "d" getrennt sind, wobei der zweite Abschnitt (5) des Gewebes (2) ferner Folgendes aufweist:
- einen dritten Bereich (5c) des Gewebes, der als Verbindungsbrücke der zwei parallelen Bereiche (5a, 5b) wirkt, um den Abstand "d" der Trennung abzudecken, wobei sich der dritte Bereich des Gewebes, der als Brücke wirkt, zwischen den zwei parallelen Bereichen erstreckt, wobei der laterale Rand desselben im Wesentlichen mit dem Rand der Enden der zwei parallelen Bereiche in Richtung des ersten Abschnittes (3) ausgerichtet ist, und wobei der erste Abschnitt (3) zum Anbringen des Gewebes an der Beckenhöhle Folgendes umfasst:
- einen Bereich (3a) des Gewebes (2) zum Befestigen an dem sakralen Promontorium, der sich im Wesentlichen mit einem der zwei parallelen Bereiche (5a, 5b) ausgerichtet und mit einem Ende desselben verbunden erstreckt, wobei der Bereich (3a) des Gewebes (2) zum Befestigen an dem sakralen Promontorium einen im Wesentlichen rechten Winkel mit dem dritten Bereich (5c) bildet, der als Brücke wirkt, wodurch es möglich ist, dass der dritte Brückenbereich (5c) das Beckenorgan (1a, 1b) teilweise und lateral umgibt, um die zwei parallelen Bereiche (5a, 5b) in dem vorderen oder hinteren Teil des Organs (1a, 1b) zu platzieren.

2. Implantat gemäß Anspruch 1, bei dem der erste und der zweite Abschnitt (3, 5) des Gewebes (2) in einem einzelnen Stück des Gewebes (2) einer im Wesentlichen flachen Struktur ohne Schweißstellen oder Verbindungsstellen zwischen den Bereichen (3,5) integriert sind.

3. Implantat gemäß Anspruch 1, bei dem die Struktur und Dichte des Gewebes (2) für alle Bereiche (3, 5a, 5b, 5c) des Implantats gleichmäßig ist.

4. Implantat gemäß einem der Ansprüche 1 bis 3, bei dem sich der Bereich des Gewebes (3a) zum Befestigen an dem Sakrum (4) von dem Ende eines der parallelen Bereiche (5a) erstreckt, wobei einer der lateralen Ränder (6) desselben im Wesentlichen mit dem lateralen Rand (7) des Bereiches (5a), mit dem derselbe verbunden ist, ausgerichtet gehalten wird.

5. Implantat gemäß einem der vorhergehenden Ansprüche, bei dem der Abstand "d" der Trennung oder die Breite (A_{5c}) des dritten Bereiches, der als Brücke (5c) wirkt, derart dimensioniert ist, das Beckenorgan (1b) lateral zu umgeben, um die sichere und angenehme Behandlung eines vaginalen (1b) Prolapses zu ermöglichen, die den Uterus (1a) schont.

6. Implantat gemäß Anspruch 5, bei dem der Abstand "d" in einem Bereich von 0,035 m und 0,045 m liegt.

7. Implantat gemäß einem der vorhergehenden Ansprüche, bei dem die zwei parallelen Bereiche (5a, 5b) des Gewebes (2) beide in der Form eines Rechteckes mit geeigneten Abmessungen (A₅ₐ, A_{5b}, h₅ₐ, h_{5b}) ausgebildet sind, um die Anatomie des vorderen oder hinteren Teils des Beckenorgans (1b) abzudecken.

8. Implantat gemäß Anspruch 7, bei dem das Beckenorgan eine Vagina (1b) ist und einer der parallelen Bereiche (5b) in der Form eines Rechteckes eine Flächengröße aufweist, die größer ist als die des anderen Bereiches (5a), um den vorderen Teil der Vagina (1b) anatomisch abzudecken.

9. Implantat gemäß Anspruch 7, bei dem der Bereich (3a) des Gewebes zum Befestigen an dem sakralen Promontorium (4) in der Form eines Rechteckes mit einer Breite (A₃ₐ) ausgebildet ist, die geringer ist als die Breite (A₅ₐ) des Bereiches (5a) des Gewebes (2) des zweiten Abschnittes (3) des Implantats, mit dem derselbe verbunden ist.

10. Implantat gemäß Anspruch 1, bei dem der Brückenbereich (5c) dazu dimensioniert und ausgebildet ist, das Beckenorgan (1b) lateral zu umgeben, um die sichere und angenehme Behandlung eines vaginalen (1b) Prolapses zu ermöglichen, die den Uterus schont (1a).

11. Implantat gemäß Anspruch 1, bei dem das Gewebe (2) ein Netz ist, vorzugsweise ein Netz aus Monofilpolypropylengarnen, die in Gruppe 1 der AMID-Klassifizierung klassifiziert sind.

12. Implantat gemäß Anspruch 11, bei dem die Poren des Netzes eine Größe gleich oder größer als 0,0010 m aufweisen.

13. Implantat gemäß einem der Ansprüche 11 bis 12, bei dem die geometrische Struktur der Poren im Wesentlichen regelmäßig ist, vorzugsweise eine geometrische Struktur in der Form eines Hexagons.

14. Implantat gemäß einem der vorhergehenden Ansprüche, bei dem das Beckenorgan eine Vagina (1b) oder ein Uterus (1a) einer Patientin ist.

15. Chirurgischer Gerätesatz, der ein Implantat gemäß einem der vorhergehenden Ansprüche, ein laparoskopisches Instrument zum Einführen des Implantats und eine Einrichtung zum Befestigen der Position des Implantats in dem Organ nach dem Platzieren aufweist, wobei die Einrichtung vorzugsweise ein biokompatibles Haftmittel umfasst, um die Bereiche (5a, 5b, 5c) des zweiten Abschnittes (5) des Implantates an dem Beckenorgan (1a, 1b) zu befestigen.

## Revendications

1. Implant chirurgical pour le traitement du prolapsus de l'organe pelvien (1a,1b) par laparoscopie et via sacrocolpopexie et/ou sacrohystéropexie, comprenant un tissu (2) de matière implantable, comprenant :
- une première portion (3) pour fixer ledit tissu sur un ou plusieurs points de la cavité pelvienne.
- une seconde portion (5) jointe à la première portion (3) pour fixer ledit tissu (2) audit organe pelvien (1a,1b), ladite seconde portion (5) de tissu (2) comprenant :
- deux sections (5a,5b) de tissu (2) pour respectivement fixer dans la partie antérieure et postérieure dudit organe pelvien (1a,1b), lesdites deux sections (5a,5b) de tissu (2) étant disposées de manière substantiellement parallèle sur le même plan et séparées l'une de l'autre d'une distance « d », ladite seconde portion (5) de tissu (2) comprenant en outre :
- une troisième section (5c) de tissu agissant comme un pont de liaison desdites deux sections parallèles (5a,5b) pour couvrir ladite distance « d » de séparation, dans laquelle ladite troisième section de tissu agissant comme un pont, s'étend entre lesdites deux sections parallèles, son bord latéral étant substantiellement aligné avec le bord des extrémités desdites deux sections parallèles, vers la première portion (3) et dans laquelle ladite première portion (3) pour fixer le tissu à la cavité pelvienne comprend :
- une section (3a) de tissu (2) devant se fixer au promontoire sacré s'étendant de manière substantiellement alignée et jointe à l'extrémité de l'une desdites deux sections parallèles (5a,5b), ladite section (3a) de tissu (2) devant se fixer au promontoire sacré formant un angle substantiellement droit avec la troisième section (5c) qui agit comme un pont, rendant possible pour ladite troisième section de pont (5c) d'entourer partiellement et latéralement ledit organe pelvien (1a,1b) afin de placer lesdites deux sections parallèles (5a,5b) dans la partie antérieure ou postérieure dudit organe (1a, 1b).

2. Implant selon la revendication 1, dans lequel lesdites première et seconde portions (3,5) du tissu (2) sont intégrées dans une seule pièce de tissu (2) de structure substantiellement plate sans soudures ni joints entre lesdites sections (3,5).

3. Implant selon la revendication 1, dans lequel la structure et la densité dudit tissu (2) est uniforme pour toutes les sections (3,5a,5b,5c) de l'implant.

4. Implant selon une quelconque des revendications 1 à 3, dans lequel ladite section de tissu (3a) devant se fixer au sacrum (4) s'étend de l'extrémité de l'une desdites sections parallèles (5a) en maintenant l'un de ses bords latéraux (6) substantiellement aligné au bord latéral (7) de la section (5a) à laquelle elle est jointe.

5. Implant selon une quelconque des revendications précédentes, dans lequel ladite distance « d » de séparation, ou la largeur (A_{5c},) de la troisième section agissant comme un pont (5c), est dimensionnée pour entourer latéralement l'organe pelvien (1b) pour permettre un traitement sûr et confortable du prolapsus vaginal (1b) préservant l'utérus (1a).

6. Implant selon la revendication 5, dans lequel ladite distance « d » est comprise entre 0,035 m et 0,045 m.

7. Implant selon une quelconque des revendications précédentes, dans lequel lesdites deux sections parallèles (5a,5b) de tissu (2) sont chacune configurée en forme de rectangle de dimensions appropriées (A₅ₐ, A_{5b}, h₅ₐ, h_{5b}) pour couvrir l'anatomie de la partie antérieure ou postérieure dudit organe pelvien (1b).

8. Implant selon la revendication 7, dans lequel ledit organe pelvien est un vagin (1b) et l'une desdites sections parallèles (5b) en forme de rectangle possède une superficie plus importante que celle de l'autre section (5a) afin de couvrir anatomiquement la partie antérieure dudit vagin (1b).

9. Implant selon la revendication 7, dans lequel ladite section (3a) de tissu devant se fixer au promontoire sacré (4) est configurée en forme de rectangle ayant une largeur (A₃ₐ) inférieure à la largeur (A₅ₐ) de la section (5a) du tissu (2) de la seconde portion (3) de l'implant auquel elle est jointe.

10. Implant selon la revendication 1, dans lequel ladite section de pont (5c) est dimensionnée et configurée pour entourer latéralement ledit organe pelvien (1b) pour permettre un traitement sûr et confortable du prolapsus vaginal (1b) préservant l'utérus (1a).

11. Implant selon la revendication 1, dans lequel ledit tissu (2) est un filet, de préférence, un filet de fils de polypropylène monofilaments classé dans le groupe I de la classification AMID.

12. Implant selon la revendication 11, dans lequel les pores dudit filet sont de taille égale ou supérieure à 0,0010 m.

13. Implant selon une quelconque des revendications 11 à 12, dans lequel la structure géométrique desdits pores est substantiellement régulière, de préférence, une structure géométrique, en forme d'hexagone.

14. Implant selon une quelconque des revendications précédentes, dans lequel ledit organe pelvien est un vagin (1b) ou un utérus (1a) d'une patiente.

15. Kit chirurgical comprenant un implant selon une quelconque des revendications précédentes, un instrument laparoscopique pour introduire l'implant et des moyens pour fixer la position de l'implant dans l'organe une fois placé, lesdits moyens comprenant de préférence un adhésif biocompatible pour fixer les sections (5a,5b,5c) de la seconde portion (5) de l'implant sur l'organe pelvien (1a,1b).
